# EUROPEAN PATENT SPECIFICATION

(11) **EP 2 979 658 B1**
(45) Date of publication and mention of the grant of the patent: **07.03.2018**
(21) Application number: 14776556.4
(22) Date of filing: 10.01.2014
(51) Int. Cl.: A61B 17/00, A61B 17/29, A61B 18/00, A61B 18/14

(54) **HIGH-FREQUENCY TREATMENT TOOL WITH TWO ROTATABLE MEMBERS AND WITH AN INSULATING CHIP**
HOCHFREQUENZBEHANDLUNGSWERKZEUG MIT ZWEI ROTIERBAREN TEILEN UND MIT EINEM ISOLIERPLÄTTCHEN
OUTIL DE TRAITEMENT À HAUTE FRÉQUENCE AVEC DEUX MEMBRES TOURNANTS ET AVEC UNE PLAQUETTE ISOLANTE

(30) Priority: 29.03.2013 US 201361806504 P
(43) Date of publication of application: 03.02.2016
(73) Proprietor: Olympus Corporation, Tokyo 192-8507 (JP)
(72) Inventor: HYODO, Ryoji, Hachioji-shi, Tokyo 192-8507 (JP); KISHI, Kosuke, Hachioji-shi, Tokyo 192-8507 (JP)
(74) Representative: Gunzelmann, Rainer
(86) International application number: PCT/JP2014/050320
(87) International publication number: WO 2014/156219

(56) References cited:
- JP-A- H11 169 381
- JP-A- 2007 330 723
- JP-A- 2009 247 696
- JP-A- 2010 268 845
- US-A- 5 241 968
- US-A- 5 275 608
- US-A1- 2003 130 565
- US-A1- 2004 102 772

## Description

### [Technical Field]

The present invention relates to a high-frequency treatment tool.

### [Background Art]

Conventionally, performing various treatments on a treatment target portion by inserting various treatment tools into a channel of an endoscope and by projecting the various treatment tools from a distal end of the endoscope has been known. For example, as a treatment for dissecting tissue, one or more functions such as marking, incision, coagulation for hemostasis and tissue grasping of a target portion may be required for a single treatment. Since one treatment tool typically fulfills only one function, when the aforementioned treatment is performed, a removing action to remove a treatment tool that is in use at present to insert another treatment tool that is to be used next needs to be performed over and over again.

In contrast, a treatment tool described in Patent Literature 1 is equipped with a pair of grasping members at a distal end thereof which are opened or closed by forward or backward movement, and a retractable acicular electrode between the pair of grasping members. With this constitution, the treatment tool described in Patent Literature 1 allows incision using the acicular electrode and tissue grasping using the grasping members to be performed by one treatment tool.

US 2004/102772 A1 concerns an apparatus and method for minimally invasive surgery using a cutting tool with rotational cutting edges. The surgical instrument includes a tube having a distal end and a cutting edge at the distal end. A handle is attached to the proximal end of the tube. Within the tube is a shaft having a longitudinally extending blade at its distal end that is adjacent to the distal end of the tube. The blade is revolvable about the longitudinal axis of the instrument to provide rotational cutting action. One or both of the blade and the cutting edge on the tube can be electrically energized to provide for electrocauterization of the body tissue being cut.

US 5 241 968 A concerns surgical instruments that are provided with distal ends having a clevis and two discrete investment cast end effectors. One end effector is stationary relative to the clevis, and the other moves or rotates about a transverse pin of the clevis. The rotating end effector includes a first hole through which the transverse pin extends, and the surgical instrument includes an actuation mechanism for causing the rotating end effector to rotate about the transverse pin. The actuation mechanism includes a push rod which is either directly coupled to the proximal end of the rotating end effector or coupled by a staple element. The stationary second end effector, while including a first hole through which the transverse pin extends, has a boss which engages a hole in the clevis and is not coupled to the actuation mechanism.

### [Citation List]

### [Patent Literature]

### [Patent Literature 1]

Japanese Unexamined Patent Application, First Publication No. H11-169381

### [Summary of Invention]

### [Technical Problem]

The treatment tool described in Patent Literature 1 allows the tissue grasping and the treatment using the acicular electrode to be performed by a single treatment tool. However, when an angle of the acicular electrode is adjusted to tissue, the entirety of an endoscope into which the treatment tool is inserted needs to be displaced, and manipulation becomes complicated. Also, since the grasping members cannot be housed in a sheath, the grasping members get in the way when a procedure using the acicular electrode is performed.

### [Solution to Problem]

A high-frequency treatment tool according to a first aspect of the present invention includes the features of claim 1.
Further aspects of the invention are described in the dependent claims.

### [Advantageous Effects of Invention]

A procedure can be performed in an easy and suitable way with the high-frequency treatment tool according to claim 1 while fulfilling a plurality of functions in the treatment part.

### [Brief Description of Drawings]

Fig. 1 is a schematic view showing a high-frequency treatment tool according to a first embodiment of the present invention.
Fig. 2 is a schematic view showing a state in which the high-frequency treatment tool according to the first embodiment of the present invention is mounted on an endoscope.
Fig. 3 is a schematic view showing a partly cut treatment part of the high-frequency treatment tool according to the first embodiment of the present invention.
Fig. 4 is a view seen from an arrow A of Fig. 3.
Fig. 5A is a view showing a storage mode of the treatment part according to the first embodiment of the present invention.
Fig. 5B is a view showing one form of treatment with the treatment part according to the first embodiment of the present invention.
Fig. 5C is a view showing one form of treatment with the treatment part according to the first embodiment of the present invention.
Fig. 6 is a view showing one form of use of the treatment part according to the first embodiment of the present invention.
Fig. 7 is a schematic view showing a partly cut treatment part of a high-frequency treatment tool according to a second embodiment of the present invention.
Fig. 8 is a view seen from an arrow B of Fig. 7.
Fig. 9 is a view showing one process in use of the high-frequency treatment tool according to the second embodiment of the present invention.
Fig. 10 is a view showing one process in use of the high-frequency treatment tool according to the second embodiment of the present invention.
Fig. 11 is a view showing one process in use of the high-frequency treatment tool according to the second embodiment of the present invention.
Fig. 12 is a view showing one process in use of the high-frequency treatment tool according to the second embodiment of the present invention.
Fig. 13 is a view showing one process in use of the high-frequency treatment tool according to the second embodiment of the present invention.
Fig. 14 is a view showing a medical manipulator to which the high-frequency treatment tool of the present invention can be applied.

### [Description of Embodiments]

### (First embodiment)

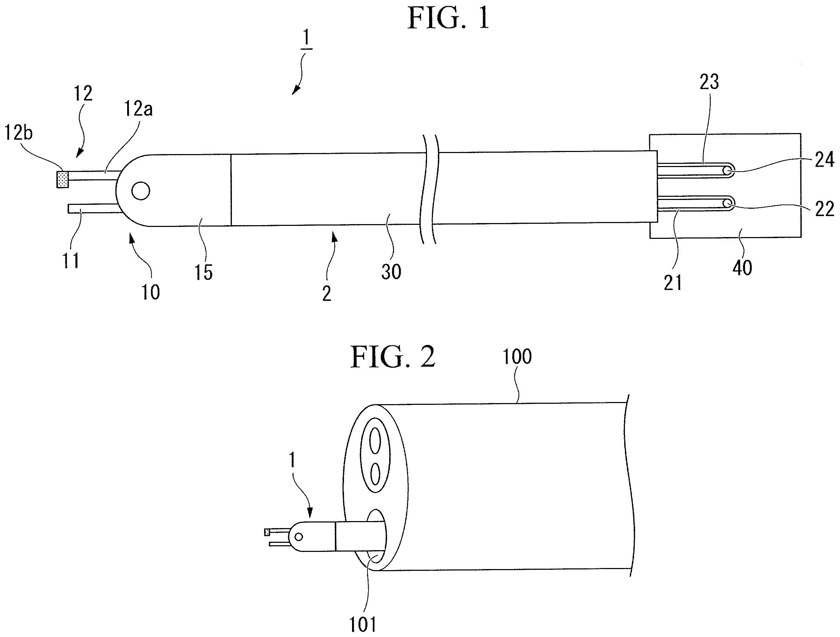

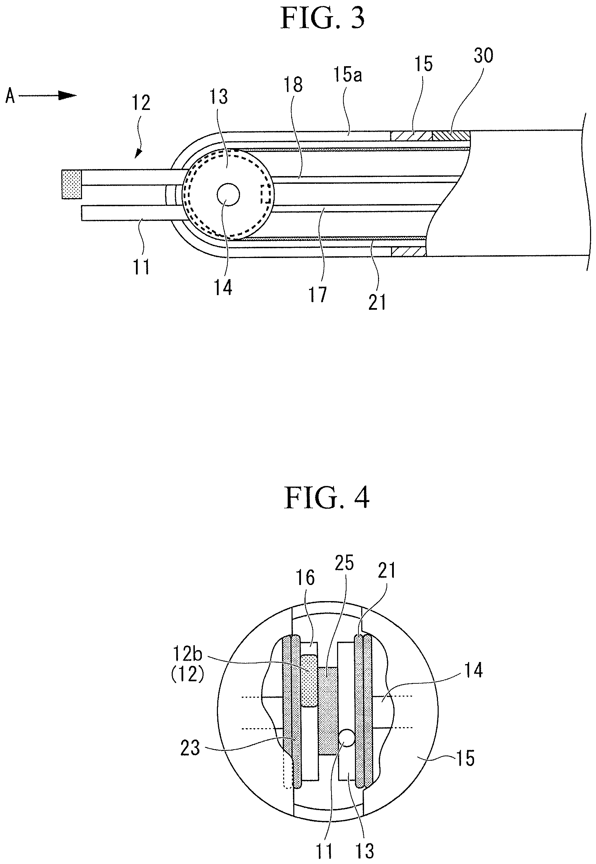

A first embodiment of a high-frequency treatment tool according to the present invention will be described with reference to Figs. 1 to 6. Fig. 1 is a schematic view showing a high-frequency treatment tool according to a first embodiment of the present invention. Fig. 2 is a schematic view showing a state in which the high-frequency treatment tool according to the present embodiment is mounted in an endoscope. Fig. 3 is a schematic view showing a partly cut treatment part of the high-frequency treatment tool according to the present embodiment. Fig. 4 is a view seen from an arrow A of Fig. 3.

As shown in Fig. 1, the high-frequency treatment tool 1 is equipped with an insertion part 2 and a treatment part 10 that are insertable into a human body, and a drive assembly (manipulation part) 40. The treatment part 10 is provided at a distal end side of the insertion part 2. The drive assembly 40 is connected to the insertion part 2.

The insertion part 2 has an elongate shape with which it can be inserted into an interior of, for instance, a forceps channel 101 at an endoscope 100 (see Fig. 2) or another known manipulator. As shown in Fig. 1, the insertion part 2 is equipped with a flexible tube part 30. Hereinafter, a side at which the treatment part 10 is provided is referred to as a distal end of the insertion part 2, and a side opposite to the side at which the treatment part 10 is provided is referred to as a proximal end of the insertion part 2.

The treatment part 10 is equipped with a cover member 15, a first knife (first treatment member) 11, and a second knife (second treatment member) 12. The cover member 15 is mounted on the distal end of the insertion part 2. The first and second knives 11 and 12 are supported on the cover member 15 such that rotating manipulations can be performed independently of each other. The first knife 11 is formed of, for example, a metal in a rod shape. The second knife 12 is equipped with a main body 12a and an insulating chip 12b, and a distal end thereof is insulated. The main body 12a has the same material and shape as the first knife 11. The insulating chip 12b is mounted on the distal end of the main body 12a. The first knife 11 and the second knife 12 are connected to separate high-frequency power supplies (not shown) by electric wires 17 and 18 shown in Fig. 3, and are supplied with high-frequency currents, thereby functioning as known high-frequency knives.

As shown in Fig. 3, the first knife 11 is mounted on a first pulley 13. The first pulley 13 is supported on a rotating shaft 14. The rotating shaft 14 is supported on the cover member 15. The cover member 15 is mounted on a distal end of a flexible tube part 30 and covers a proximal end side of the treatment part 10. In a state in which a
first wire (first transmission member) 21 is wound around the first pulley 13 one turn or more, a part thereof is fixed. As shown in Fig. 1, the first wire 21 passes through the flexible tube part 30, protrudes from a proximal end side of the flexible tube part 30, and is wound around a first drive shaft 22. The first drive shaft 22 is provided for a drive assembly 40, and is connected to a drive shaft of a drive mechanism such as a motor (not shown). If the drive mechanism is driven, the first drive shaft 22 is rotated, and the first wire 21 moves. As a result, the first pulley 13 and the first knife 11 mounted on the first pulley 13 are rotated about the rotating shaft 14.

The second knife 12 is mounted on a second pulley 16. The second pulley 16 is disposed on approximately the same axis as the first pulley 13. In the same way as the first wire 21, a second wire (second transmission member) 23 is wound around and fixed to the second pulley 16. In the same way as the first wire 21, the second wire 23 is wound around a second drive shaft 24 provided for the drive assembly 40. The second wire 23 can rotate the second pulley 16 and the second knife 12 by rotating the second drive shaft 24.

As shown in Fig. 4, an insulating member 25 is disposed between the first pulley 13 and the second pulley 16. An electric short-circuit between the first knife 11 and the second knife 12 through the first pulley 13 and the second pulley 16 is prevented by the insulating member 25. Also, the rotating shaft 14 is inserted into the first pulley 13 and the second pulley 16. The rotating shaft 14 is also configured so as not to cause the aforementioned electric short-circuit, for example, by forming it of an insulating material or performing insulating coating on an outer surface thereof.

The first and second wires 21 and 23 may be formed by suitably selecting a known material such as a metal or a resin. The first and second wires 21 and 23 may be composed of the same material or different materials.

An operation when the high-frequency treatment tool 1 configured as described above is used will be described.

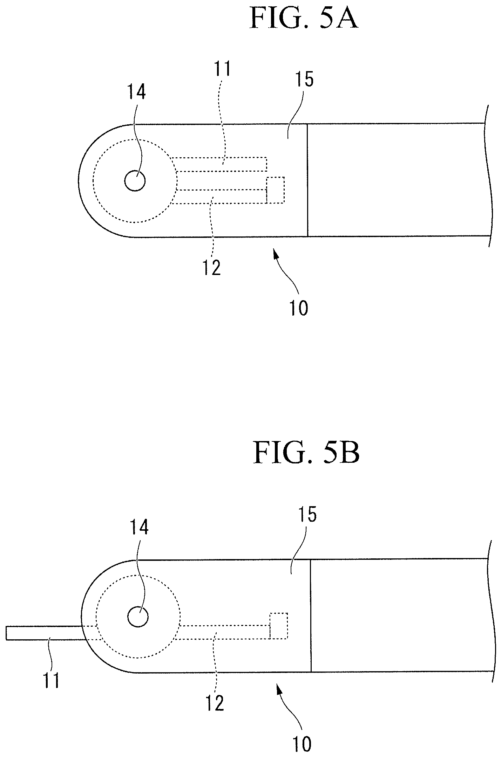

If the first and second drive shafts 22 and 24 of the drive assembly 40 are rotated via the drive mechanism, the first and second pulleys 13 and 16 can be rotated about the shaft 14. Thereby, if the first knife 11 is displaced to be separated from the second knife 12 in the state shown in Fig. 3, the first knife 11 is evacuated such that a distal end thereof becomes a proximal end side relative to the rotating shaft 14. Further, the first knife 11 enters from a slot 15a provided in the cover member 15 into the cover member 15, and is housed in the cover member 15 as shown in Fig. 5A. Similarly, the second knife 12 can also be housed in the cover member 15. Hereinafter, as in Fig. 5A, the state in which the first knife 11 and the second knife 12 are housed in the cover member 15 is referred to as storage mode.

If only one of the first knife 11 and the second knife 12 is evacuated, a procedure such as incision can be performed using only the other high-frequency knife protruding from the cover member 15. Also, if the first knife 11 and the second knife 12 are not supplied with high-frequency currents, they can be used as pressing rods for dissecting the tissue.

If the first knife 11 and the second knife 12 are operated in cooperation with each other, the tissue or the like can be sandwiched and gripped between the first knife 11 and the second knife 12. At this time, if the first knife 11 and the second knife 12 are not supplied with the high-frequency currents, the treatment part 10 may function as common grasping forceps. If the currents are applied to the first and second knives 11 and 12, the treatment part 10 may function as coagulating forceps that apply heat to the sandwiched tissue.

Next, an example of a procedure when performing endoscopic submucosal dissection (ESD) using the endoscope 100 and the high-frequency treatment tool 1 will be described.

First, an operator inserts the endoscope 100 into the mouth of a patient, and advances a distal end of the endoscope 100 to the vicinity of a treatment target portion. Next, physiological saline is injected under the treatment target portion to distend tissue, and the target portion to be excised is separated from other tissues.

Next, the treatment part 10 of the high-frequency treatment tool 1 is set in the storage mode, is inserted into the forceps channel 101 from, for instance, a forceps port of the proximal end side of the endoscope 100, and protrudes from a distal end of the endoscope 100. When an insertion part of the endoscope 100 is soft, the forceps channel 101 also frequently meanders in a body of the patient. However, in the high-frequency treatment tool 1 according to the present embodiment, since the treatment part 10 is set in the storage mode and is inserted into the forceps channel 101, the first knife 11 and the second knife 12 can be easily inserted without damaging an inner wall of the forceps channel 101.

While observing the target portion with the endoscope 100, the operator manipulates the drive assembly 40 to rotate the first pulley 13, and has only the first knife 11 protrude from the cover member 15 as shown in Fig. 5B. In a state in which a current is applied to the first knife 11, the distal end of the first knife 11 comes into contact with the tissue, and the tissue is cauterized in a point shape and marked. Afterward, an incision is slowly made in the target portion by the first knife 11.

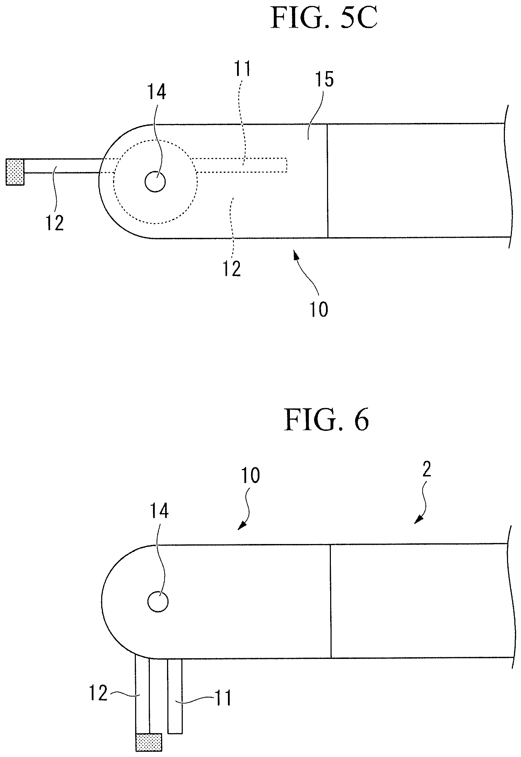

After the incision is made to some extent, the operator puts the first knife 11 into the cover member 15, and has the second knife 12 protrude from the cover member 15 as shown in Fig. 5C. Then, resection of the target portion is conducted using the second knife 12. Since the distal end of the second knife 12 is provided with the insulating chip 12b, even if the distal end of the second knife 12 is exposed to the tissue, a resection procedure can be accurately performed without cauterizing the tissue.

When bleeding occurs during resection, the first and second knives 11 and 12 may be used as the coagulating forceps as needed. Also, in a state in which the excised tissue is gripped by the first and second knives 11 and 12, the high-frequency treatment tool 1 is removed, and the tissue may be collected.

In each process of the aforementioned procedure, the first and second pulleys 13 and 16 are suitably rotated, and a direction in which the distal end of the treatment part 10 protrudes may be adjusted at the insertion part 2. For example, the first and second knives 11 and 12 may be inclined with respect to the axis of the insertion part 2, or perpendicular to the axis. Further, as shown in Fig. 6, even when the first and second knives 11 and 12 function as the forceps, opening/closing positions and directions of the forceps may be appropriately changed.

In the high-frequency treatment tool 1 according to the present embodiment, the first and second knives 11 and 12 of the treatment part 10 can be rotated about the shaft 14. For this reason, angle adjustment can be performed without moving the endoscope 100 into which the high-frequency treatment tool 1 is inserted. That is, the shaft 14 functions as a joint for the angle adjustment. Further, one of the first knife 11 and the second knife 12 is rotated and evacuated, and thereby does not get in the way when a procedure is performed by the other. As a result, the procedure can be performed in an easy and suitable way while numerous functions are fulfilled in the treatment part 10.

Also, the aforementioned evacuating movement and the joint driving of each knife are substantially the same movement. For this reason, both of the aforementioned evacuating movement and the joint driving of each knife can be conducted by the first and second wires 21 and 23 Therefore, even if the complicated movement of the treatment part 10 is possible, a structure in which an increase in the size of a device is limited can be achieved.

In addition, even when the treatment part 10 is inserted into a channel of a soft manipulator that is apt to meander, the treatment part 10 is used in the storage mode, damage to an inner wall of the channel or a catch to the inner wall is limited, and the treatment part 10 can be easily inserted.

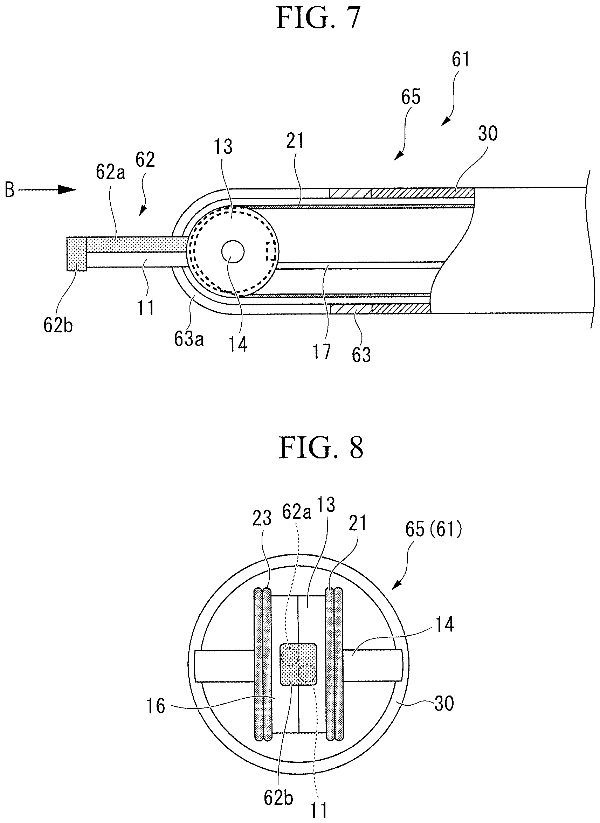

Next, a second embodiment of the present invention will be described with reference to Figs. 7 to 13. In the following description, the same components as those that have already been described are given the same reference signs, and a duplicate description thereof will be omitted here.

Fig. 7 is a view showing a partly cut distal end side of a high-frequency treatment tool 61 according to the present embodiment. Fig. 8 is a view seen from an arrow B of Fig. 7. Fig. 8 shows a structure of a treatment part 65 in which a cover member is excluded such that the structure can be more easily understood.

As shown in Fig. 7, the high-frequency treatment tool 61 has a second member 62 mounted on a second pulley 16 instead of the second knife 12. The second member 62 has a main body 62a and a distal end chip 62b that have the same shapes as the main body and the insulating chip at the second knife, respectively. The main body 62a and the distal end chip 62b are formed of an insulator, or surfaces thereof are coated by an insulating layer. Thereby, the main body 62a and the distal end chip 62b are formed to have an insulating property. No electric wire is connected to the second member 62, and no high-frequency current is supplied to the second member 62.

Since there is no need to secure insulation of the first knife 11 and the second member 62, no insulating member is disposed between a first pulley 13 and the second pulley 16 as shown in Fig. 8.

If a first wire 21 and a second wire 23 are manipulated to bring the first knife 11 and the second member 62 close to each other, the distal end chip 62b of the second member 62 covers a distal end of the first knife 11 as shown in Fig. 7. In this state, the first knife 11 and the second member 62 are displaced in one body while the first knife 11 is supplied with a high-frequency current. Thereby, the first knife 11 and the second member 62 can function as a high-frequency knife whose distal end is insulated.

A basic shape of the cover member 63 is an approximately cylindrical shape as in the first embodiment. However, the cover member 63 has a smaller dimension in an axis direction than the cover member 15 of the first embodiment, and has a shape in which a slot 63a thereof is also shorter than that of the cover member 15. For this reason, the first knife 11 and the second member 62 have structures in which any one thereof can be evacuated up to a position at which the one does not get in the way of the procedure using the other, but cannot be housed in the cover member 63.

An operation when the high-frequency treatment tool 61 is used will be described taking the case of performing ESD as an example. First, like the first embodiment, a distal end of an endoscope 100 is advanced up to the vicinity of a treatment target portion, and the treatment target portion is distended.

Next, the high-frequency treatment tool 61 is inserted into the endoscope 100. At this time, since the treatment part 65 of the high-frequency treatment tool 61 cannot be in a storage mode, the first knife 11 and the second member 62 are inserted in a state in which they are directed forward as in Fig. 7.

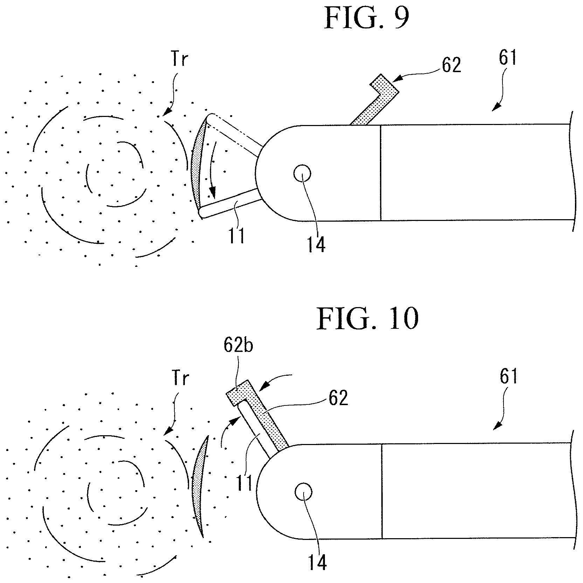

After the high-frequency treatment tool 61 is projected from the distal end of the endoscope 100, an operator rotates and evacuates the second member 62 as shown in Fig. 9. Afterward, the operator performs a marking or an incision of target portion Tr using the first knife 11. In Figs. 9 to 11, a state in which the distended target portion Tr is viewed from above is shown. In Fig. 9, an example in which an incision is made by rotating the first knife 11 about the rotating shaft 14 is illustrated. However, as in the related art, the incision may be made by displacing the entirety of the endoscope 100 into which the high-frequency treatment tool 61 is inserted.

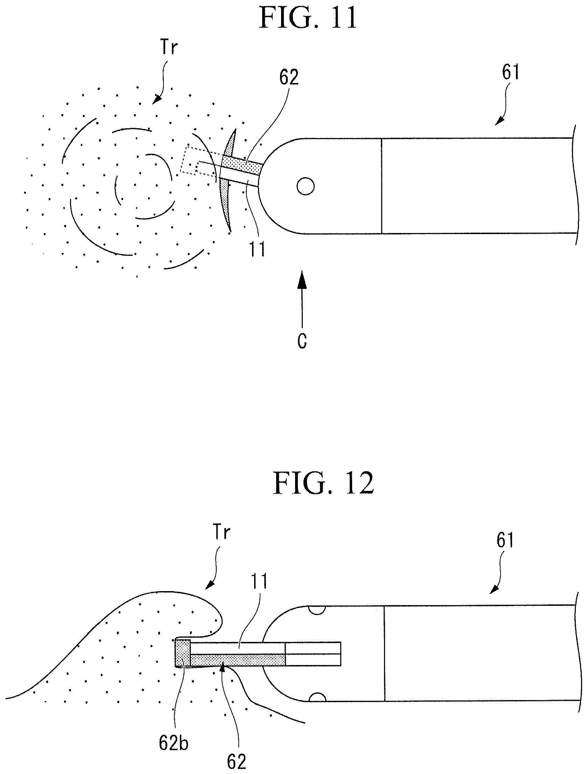

Formation of the incision triggering resection is completed, and then the operator rotates or manipulates the first knife 11 and the second member 62 to cover the distal end of the first knife 11 with the distal end chip 62b as shown in Fig. 10. As shown in Fig. 11, the operator penetrates the incision with the first knife 11 and the second member 62 functioning as the knife whose distal end is insulated, and proceeds to resection of the target portion Tr. Fig. 12 is a view seen from an arrow C of Fig. 11. As shown in Fig. 12, the distal end of the first knife 11 is insulated and covered by the distal end chip 62b. For this reason, for example, even when the high-frequency treatment tool 61 moves forward unintentionally, the target portion Tr is not excessively incised, and no perforation occurs.

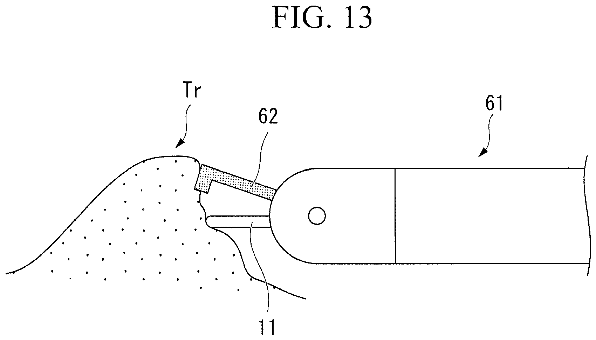

In addition, as shown in Fig. 13, while a part of the target portion Tr is being excluded by the second member 62, the incision and resection can also be performed by the first knife 11.

Even in the high-frequency treatment tool 61 according to the present embodiment, like the first embodiment, the easy and suitable procedure can be performed.

Also, since the first knife 11 can also be used as the knife whose distal end is insulated, there is no need to consider, for instance, insulation of the first pulley 13 and the second pulley 16. For this reason, a structure of the treatment part can be simplified and used as a structure in which miniaturization is easier.

Although embodiments of the present invention have been described, the present invention is not limited to the above embodiments.

For example, in the high-frequency treatment tool of the present invention, an electrical type in functioning as the high-frequency knife includes any one of a monopolar type and a bipolar type.

The high-frequency treatment tool is configured such that, for instance, in the knife to which the current is applied, the current is not automatically applied in the event of the evacuation or storage, and thereby manipulability of the high-frequency treatment tool can be improved. For example, the high-frequency treatment tools may be configured such that, for instance, a contact with an electric wire for supplying power is provided on an outer circumferential surface of the pulley for which the knife is provided, for example, the contact comes into contact with the electric wire within a predetermined rotating range, and does not come into contact with the electric wire beyond the predetermined rotating range.

In the high-frequency treatment tool, the electric wire whose distal end is formed in a ring shape may be locked on the rotating shaft, and the contact with the electric wire may be provided on an axial end face of the pulley. Thereby, in the high-frequency treatment tool, since the contact is not in contact with the electric wire when located in a ring formed at the distal end of the electric wire, an angle range of the treatment part capable of supplying power can be adjusted by the ring shape of the distal end of the electric wire.

The high-frequency treatment tool may be configured by providing a known water supply mechanism in the cover member such that the tissue attached to, for instance, the housed knife can be cleansed.

The high-frequency treatment tool may be configured by providing a handle or a dial knob with which the treatment part is manually driven on the drive shaft of the manipulation part.

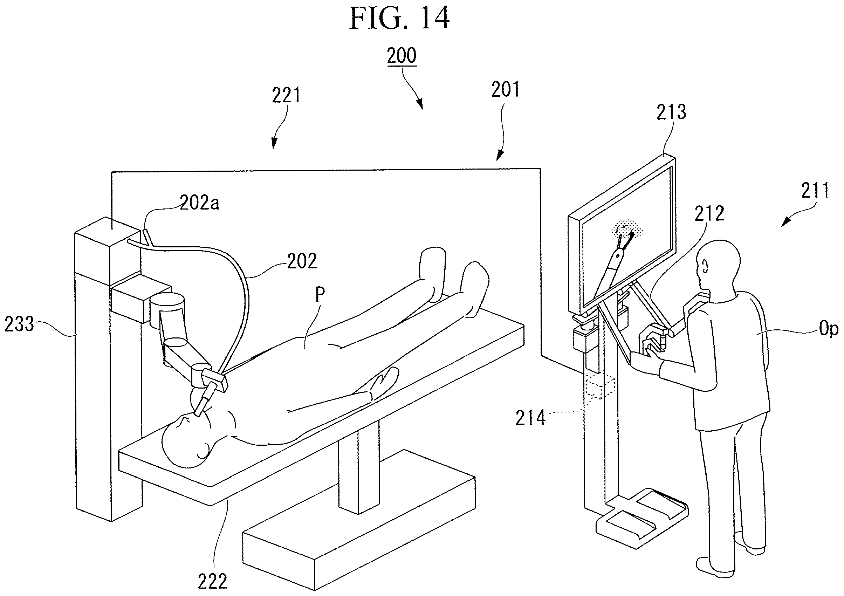

A target to which the high-frequency treatment tool of the present invention is applicable is not limited to the aforementioned endoscope. For example, a medical system 200 as shown in Fig. 14 may be configured by combining the high-frequency treatment tool with a master-slave type medical manipulator 201 having a soft insertion part 202. The medical manipulator 201 shown in Fig. 14 is equipped with a master manipulator 211 and a slave manipulator 221. The master manipulator 211 is manipulated by an operator Op. The slave manipulator 221 is provided with the insertion part 202 having an observation mechanism.

The master manipulator 211 is equipped with a master arm 212, a display unit 213, and a control unit 214. The operator Op performs manipulation input using the master arm 212. The display unit 213 displays an image recorded using the observation mechanism of the insertion part 202. The control unit 214 generates a manipulating instruction for operating the slave manipulator 221 based on movement of the master arm 212.

The slave manipulator 221 has a placement table 222 on which a patient P is placed, a polyarticular robot 223, and the insertion part 202. The polyarticular robot 223 is disposed adjacent to the placement table 222. The insertion part 202 is mounted on the polyarticular robot 223. The polyarticular robot 223 and the insertion part 202 are operated according to a manipulation instruction sent from the master manipulator 211. The high-frequency treatment tool according to the present embodiment is inserted into an insertion hole 202a provided in a proximal end of the insertion part 202, and the manipulation part of the high-frequency treatment tool is mounted at a predetermined part of the polyarticular robot 223. Thereby, the treatment part of the high-frequency treatment tool can be manipulated using the master arm 212.

The high-frequency treatment tool can obtain more merits in combination with the manipulator equipped with the soft insertion part because of an advantage that the storage mode is given, and miniaturization of the treatment part is easy. In addition, the high-frequency treatment tool can also be combined with a manipulator equipped with an insertion part having no flexibility.

Although embodiments of the present invention have been described above in detail with reference to the drawings, the specific constitution is not limited to these embodiments. The invention is defined by the appended claims.

### [Industrial Applicability]

Using the high-frequency treatment tool, the procedure can be performed in an easy and suitable way while fulfilling a plurality of functions in the treatment part.

### [Reference Signs List]

- 1:: high-frequency treatment tool
- 2:: insertion part
- 10:: treatment part
- 40:: drive assembly (manipulation part)
- 15:: cover member
- 11:: first knife (first treatment member)
- 12:: second knife (second treatment member)
- 12a, 62a:: main body
- 12b, 62b:: insulating chip
- 21:: first wire (first transmission member)
- 22:: first drive shaft
- 23:: second wire (second transmission member)
- 24:: second drive shaft

## Claims

1. A high-frequency treatment tool (1; 61) comprising:
a longitudinal insertion part (2); a manipulation part (40) which is coupled to the insertion part (2); a treatment part (10; 65) which is provided at a distal end portion of the insertion part (2), whereby
the treatment part (10; 65) includes:
a cover member (15) which is mounted on the distal end portion of the insertion part (2);
a first treatment member (11) which is rotatably supported within the cover member (15), which is configured to rotate about a shaft (14), and to which a high-frequency current is capable of being applied; and
a second member (12; 62) which is configured to rotate about the shaft (14), the second member (12; 62) including a rod-shaped main body (12a; 62a), and
the manipulation part (40) includes:
a first drive shaft (22) that is connected to the first treatment member (11) by a first transmission member (21) and rotatably driven to rotate the first treatment member (11); and
a second drive shaft (24) that is connected to the second member (12; 62) by a second transmission member (23) and rotatably driven to rotate the second member (12; 62), **characterized in that** the second member is rotatably supported within the cover member (15) and **in that** the second member includes an insulating chip (12b; 62b) mounted on a distal end of the main body (12a; 62a).

2. The high-frequency treatment tool (1; 61) according to claim 1, wherein the first treatment member (11) and the second member (12; 62) are rotatable to enter the cover member (15) to assume storage positions.

3. The high-frequency treatment tool (1; 61) according to claim 2, wherein the high-frequency treatment tool (1; 61) is configured to interrupt a supply of the high-frequency current when the first treatment member (11) is stored.

4. The high-frequency treatment tool (61) according to claim 1, wherein:
the main body (62a) of the second member (62) is formed of an insulating material; and
a distal end portion of the first treatment member (11) is configured to be covered by the insulating chip (62b) in a state where the first treatment member (11) and the second member (62) approach each other.

## Patentansprüche

1. Hochfrequenzbehandlungswerkzeug (1; 61) umfassend:
ein longitudinales Einführungsteil (2);
ein Manipulationsteil (40), welches mit dem Einführungsteil (2) gekoppelt ist;
ein Behandlungsteil (10; 65), welches an einem distalen Endteil des Einführungsteils (2) bereitgestellt ist, wobei
das Behandlungsteil (10; 65) enthält:
ein Abdeckelement (15), welches an dem distalen Endteil des Einführungsteils (2) angebracht ist;
ein erstes Behandlungselement (11), welches in dem Abdeckelement (15), welches konfiguriert ist, um sich um eine Achse (14) zu drehen, drehbar gelagert ist, und an welchen ein Hochfrequenzstrom angelegt werden kann; und
ein zweites Element (12; 62), welches eingerichtet ist, um sich um die Achse (14) zu drehen, wobei
das zweites Element (12; 62) einen stabförmigen Hauptkörper (12a; 62a) enthält, und
das Manipulationsteil (40) enthält:
eine erste Antriebsachse (22), die durch ein erstes Übertragungselement (21) mit dem ersten Behandlungselement (11) verbunden ist und drehbar angetrieben wird, um das erste Behandlungselement (11) zu drehen; und
eine zweite Antriebsachse (24), die durch ein zweites Übertragungselement (21) mit dem zweiten Behandlungselement (12; 62) verbunden ist und drehbar angetrieben wird, um das zweite Behandlungselement (12; 62) zu drehen, **dadurch gekennzeichnet, dass** das zweite Element in dem Abdeckelement (15) drehbar gelagert ist, und dass das zweite Element ein Isolierplättchen (12b; 62b) enthält, welches an einem distalen Ende des Hauptkörper (12a; 62a) angebracht ist.

2. Hochfrequenzbehandlungswerkzeug (1; 61) gemäß Anspruch 1, wobei das erste Behandlungselement (11) und das zweite Behandlungselement (12; 62) drehbar sind, um in das Abdeckelement (15) einzutreten, um Lagerpositionen einzunehmen.

3. Hochfrequenzbehandlungswerkzeug (1; 61) gemäß Anspruch 2, wobei das Hochfrequenzbehandlungswerkzeug (1; 61) eingerichtet ist, um eine Versorgung des Hochfrequenzstroms zu unterbrechen, wenn das erste Behandlungselement (11) eingelagert ist.

4. Hochfrequenzbehandlungswerkzeug (61) gemäß Anspruch 1, wobei:
der Hauptkörper (62a) des zweiten Elementes (62) aus einem isolierenden Material gebildet ist; und
ein distales Endteil des ersten Behandlungselementes (11) eingerichtet ist, um von dem Isolierplättchen (62b) abgedeckt zu sein, in einem Zustand, bei dem sich das erste Behandlungselement (11) und das zweite Element (62) gegenseitig annähern.

## Revendications

1. Outil de traitement à haute fréquence (1 ; 61) comprenant :
une partie d'insertion longitudinale (2) ;
une partie de manipulation (40) qui est reliée à la partie d'insertion (2) ;
une partie de traitement (10 ; 65) qui est prévue au niveau d'une partie d'extrémité distale de la partie d'insertion (2), moyennant quoi
la partie de traitement (10 ; 65) comprend :
un élément de recouvrement (15) qui est monté sur la partie d'extrémité distale de la partie d'insertion (2) ;
un premier élément de traitement (11) qui est supporté de manière rotative dans l'élément de recouvrement (15), qui est configuré pour tourner autour d'un arbre (14), et auquel on peut appliquer un courant à haute fréquence ; et
un second élément (12 ; 62) qui est configuré pour tourner autour de l'arbre (14),
le second élément (12 ; 62) comprenant un corps principal en forme de tige (12a ; 62a),
et
la partie de manipulation (40) comprend :
un premier arbre d'entraînement (22) qui est relié au premier élément de traitement (11) par un premier élément de transmission (21) et entraîné de manière rotative pour faire tourner le premier élément de traitement (11) ; et
un second arbre d'entraînement (24) qui est relié au second élément (12 ; 62) par un second élément de transmission (23) et entraîné de manière rotative pour faire tourner le second élément (12 ; 62), **caractérisé en ce que** le second élément est supporté de manière rotative dans l'élément de recouvrement (15) et **en ce que** le second élément comprend une puce isolante (12b ; 62b) montée sur une extrémité distale du corps principal (12a ; 62a).

2. Outil de traitement à haute fréquence (1 ; 61) selon la revendication 1, dans lequel le premier élément de traitement (11) et le second élément (12 ; 62) peuvent être tournés pour entrer dans l'élément de recouvrement (15) afin de prendre des positions de stockage.

3. Outil de traitement à haute fréquence (1 ; 61) selon la revendication 2, dans lequel l'outil de traitement à haute fréquence (1 ; 61) est configuré pour interrompre l'alimentation du courant à haute fréquence lorsque le premier élément de traitement (11) est stocké.

4. Outil de traitement à haute fréquence (61) selon la revendication 1, dans lequel :
le corps principal (62a) du second élément (62) est formé d'un matériau isolant ; et
une partie d'extrémité distale du premier élément de traitement (11) est configurée pour être recouverte par la puce isolante (62b) dans un état où le premier élément de traitement (11) et le second élément (62) se rapprochent l'un de l'autre.
